# EUROPÄISCHE PATENTANMELDUNG

(11) **EP 0 741 126 A2**
(43) Veröffentlichungstag der Anmeldung: **06.11.1996**
(21) Anmeldenummer: 96106586.9
(22) Anmeldetag: 26.04.1996
(51) Int. Cl.: C07C 251/66, C08F 20/34

(54) **Verfahren zur Herstellung von Oximmethacrylaten**

(30) Priorität: 05.05.1995 DE 19516470
(71) Anmelder: Röhm GmbH, 64293 Darmstadt (DE)
(72) Erfinder: Knebel, Joachim, Dr., 64293 Darmstadt (DE); Gräff, Günther, 64665 Alsbach-Hähnlein (DE); Fölsch, Karl Josef, Dr., 55128 Mainz (DE)

(57) **Zusammenfassung**

Die Erfindung betrifft ein Verfahren zur Herstellung von Methacryloylestern von Ketoximen der allgemeinen Formel I 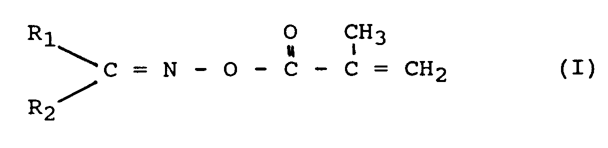 worin
- R₁: einen Alkylrest mit 1 bis 4 Kohlenstoffatomen oder
- R₂: einen Alkylrest mit 1 bis 4 Kohlenstoffatomen bedeuten oder worin
- R₁ und R₂: zusammen mit dem Keto-Kohlenstoffatom einen 5 bis 12-gliedrigen alicyclischen Ring bilden,
wobei man ein Ketoxim der allgemeinen Formel II 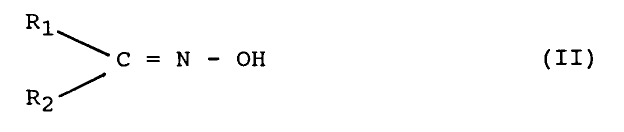 worin R₁ und R₂ die angegebenen Bedeutungen besitzen mit Methacrylsäureanhydrid umsetzt.

## Beschreibung

### Gebiet der Erfindung

Die Erfindung betrifft ein Verfahren zur Herstellung von Methacryloylestern von Ketoximen, durch Umsetzung der Ketoxime mit Methacryloylanhydrid und Verwendung der Ester als Monomere mit vernetzenden Eigenschaften.

### Stand der Technik

Oxime lassen sich mit Säurehalogeniden, insbesondere Säurechloriden acylieren, wobei in der Regel ein Säureakzeptor angewendet wird. (Vgl. Houben-Weyl, Methoden der Organischen Chemie, 3. Auflage, Bd. 10/4, S. 180, 184, G.Thieme Verlag 1968). Die acylierten Ketoxime reagieren relativ leicht weiter unter BECKMANN-Umlagerung, insbesondere wenn der Acylrest eine gute "Leaving Group" darstellt. Bei Aldoximen führt die Acylierung in der Tendenz zu den Nitrilen. Die Acetylierung von Ketoximen gelingt auch mit Acetanhydrid wenn entsprechend schonende Bedingungen eingehalten werden, beispielsweise bei Einhaltung eines neutralen pH-Werts und (Um)kristallisation bei Raumtemperatur (vgl. Houben-Weyl, Bd. 10/4 loc.cit. S. 228, 229).
In der Literatur haben insbesondere die (Meth)acrylate der Oxime von halogenierten Carbonylverbindungen Beachtung gefunden, da sie fungizide Wirkung besitzen. Die Methacryloylverbindungen aromatischer Oxime haben als Photoinitiatoren Anwendung gefunden.

Es wurde nun gefunden, daß sich nach dem erfindungsgemäßen Verfahren die Methacryloylverbindungen von Ketoximen besonders einfach und günstig herstellen lassen.
Die Erfindung betrifft somit ein Verfahren zur Herstellung der Methacryloylester von Ketoximen der allgemeinen Formel I 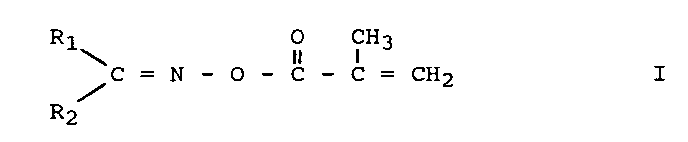 worin
- R₁: einen Alkylrest mit 1 bis 4 Kohlenstoffatomen und
- R₂: einen Alkylrest mit 1 bis 4 Kohlenstoffatomen bedeuten, oder worin
- R₁ und R₂: zusammen mit dem Ketokohlenstoffatom einen 5 bis 12-gliedrigen, alicyclischen Ring bilden.
durch Umsetzung eines Ketoxims der allgemeinen Formel II 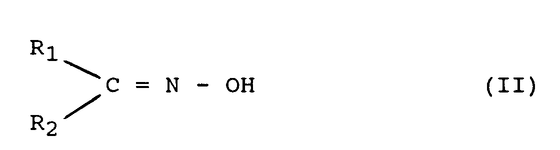 worin R₁ und R₂ die oben angegebenen Bedeutungen besitzen mit Methacrylsäureanhydrid vorzugsweise in äquimolaren Mengen und bei Raumtemperatur. Dabei bildet sich jeweils ein Mol Methacrylsäure.

Besonders genannt sei der Fall, worin R₁ und R₂ für Methyl steht (Acetonoxim) oder worin R₁ und R₂ zusammen mit dem Ketokohlenstoffatom einen 6-gliedrigen aliphatischen Ring bilden (Cyclohexanonoxim).

### Ausführung der Erfindung

Die zur Durchführung des erfindungsgemäßen Acylierungsverfahrens benötigten Verbindungen, die Ketoxime der Formel II und das Methacrylsäureanhydrid sind bekannt (vgl. Houben-Weyl, Bd. 10/4, loc.cit. S. 17 - 28, H. Rauch-Puntigam, Th. Völker, Acryl- und Methacrylverbindungen, S. 43, Springer-Verlag 1967). Die Herstellung der Methacryloylester kann in einfachster Weise durch Vereinigen der Komponenten in einem geeigneten Reaktionsgefäß, beispielsweise einem Rundkolben unter Abführen der Reaktionswärme erfolgen.

Hervorzuheben ist, daß die Umsetzung bei Raumtemperatur und mit äquimolaren Mengen an beiden Edukten durchgeführt werden kann. Die Reinigung kann in an sich bekannter Weise, z.B. durch Vakuumfraktionierung erfolgen. So kann das Acetonoximmethacrylat durch Vakuumfraktionierung bei 100^{o}/1,4 mbar gereinigt werden.
Der Cyclohexanonoximester wird vorteilhaft durch Abziehen der Methacrylsäure im Vakuum (ca. 2 mbar) bei Badtemperaturen um 100 Grad C aufgearbeitet. Es muß als überraschend gelten, daß die Umsetzung trotz der Bildung von Methacrylsäure und des schließlich noch spurenweise vorhandenen Methacrylsäureanhydrids in hervorragender Ausbeute und Reinheit gelingt. Insbesondere ist hervorzuheben, daß in der Regel die BECKMANN-Umlagerung nahezu keine Rolle spielt.

Für die Methacryloylester von Ketoximen der Formel I eröffnen sich als radikalisch polymerisierbare und vernetzungsfähige Monomere vielfältige Anwendungsmöglichkeiten.
Beispielsweise können sie in Emulsionspolymerisate eingebaut werden, vorzugsweise zusammen mit einem Monomeren mit reaktiver Carbonylfunktion.
Erwähnt sei unter diesem Aspekt ein Emulsionspolymerisat aus mindestens einem Monomeren der Formel I neben Methylmethacrylat und Butylacrylat, in das gleichzeitig 2-Acetoacetoxyethylmethacrylat eingebaut wurde. Gießfolien aus derartigen Polymerdispersionen vernetzen unter milden Bedingungen - beispielsweise bei 40 Grad C und zeigen geringe Quellung und wenig Gewichtsverlust beim Behandeln mit Lösungsmitteln.

Die folgenden Beispiele dienen zur Erläuterung der Herstellung der Verbindungen der Formel I.

### BEISPIELE

### Beispiel 1

### Herstellung von Acetonoximmethacrylat

In einem 1 l-Vierhalsrundkolben mit Lufteinleitrohr, mechanischem Rührer, Tropftrichter, Thermometer und Kühler legt man 281 g (4,3 mol) Acetonoxim vor. Unter Luftzufuhr und Rühren wird in ca. 1,5 Stunden 594 g (3,8 mol) Methacrylsäureanhydrid bei 25 Grad C zugetropft (bei Überschreiten der Temperatur wird mit Eiswasser gekühlt). Man läßt 20 min bei Raumtemperatur nachreagieren, versetzt das Reaktionsprodukt mit 0,136 g Hydrochinonmonomethylether sowie 88 g Lipinol (Siedehilfsmittel, Wz. der Hüls AG) und fraktioniert im Vakuum durch eine 20 cm lange verspiegelte Kolonne (⌀ : 25 mm), die mit 6 x 6 mm² Glasraschigringen gefüllt ist. Nach 530 g Vorlauf (47 - 84 Grad C / 1,4 - 1,8 mbar), die das Produkt nach gaschromatographischer Untersuchung zu 40 % enthalten, isoliert man bei 84 - 86 Grad C (1,4 - 1,8 mbar) 252 g (42,3 % d.Th) Acetonoximmethacrylat als leicht gelb gefärbte Flüssigkeit, die nach der GC-Analyse eine Reinheit von 90 % besitzt und als Verunreinigungen Methacrylsäure und Methacrylsäureanhydrid aufweist.

### Beispiel 2

### Herstellung von Cyclohexanonoximmethacrylat

In einem 100 ml-Dreihalskolben mit Lufteinleitrohr, Rückflußkühler, mechanischem Rührer und Tropftrichter sowie Thermometer, legt man 20 g (0,18 mol) Cyclohexanonoxim vor. Man tropft 27 g (0,17 mol) Methacrylsäureanhydrid so zu, daß eine Innentemperatur von 30 Grad C nicht überschritten wird. Nach 4 Stunden Nachreaktion bei Raumtemperatur wird die Reaktionsmischung nach Zusatz von 0,9 g Hydrochinonmonomethylether bei 2 mbar und 100 Grad C Badtemperatur am Rotationsverdampfer eingeengt. Es verbleiben 32 g gelblicher fester Rückstand (91,5 % d.Th.), die nach GC zu 88 % aus Cyclohexanonoxiummethacrylat bestehen.

## Patentansprüche

1. Verfahren zur Herstellung von Methacryloylestern von Ketoximen der allgemeinen Formel I 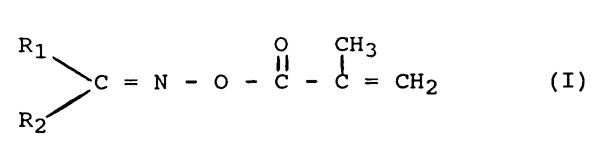 worin
R₁ einen Alkylrest mit 1 bis 4 Kohlenstoffatomen oder
R₂ einen Alkylrest mit 1 bis 4 Kohlenstoffatomen oder bedeuten oder worin
R₁ und R₂ zusammen mit dem Keto-Kohlenstoffatom einen 5 bis 12-gliedrigen alicyclischen Ring bilden,
dadurch gekennzeichnet,
daß man ein Ketoxim der allgemeinen Formel II 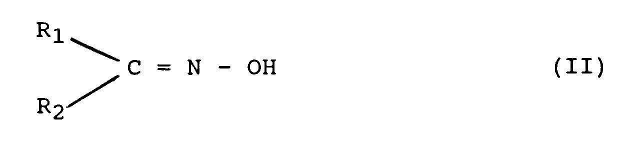 worin R₁ und R₂ die angegebenen Bedeutungen besitzen mit Methacrylsäureanhydrid umsetzt.

2. Verfahren gemäß Anspruch 1, dadurch gekennzeichnet, daß die Umsetzung mit äquimolaren Mengen an Ketoxim der allgemeinen Formel II und Methacrylsäureanhydrid durchgeführt wird.

3. Verfahren gemäß den Ansprüchen 1 und 2, dadurch gekennzeichnet, daß die Umsetzung bei Raumtemperatur durchgeführt wird.

4. Acetonoximmethacrylsäureester.

5. Cyclohexanonoximmethacrylsäureester

6. Verwendung der Methacryloylester von Ketoximen der allgemeinen Formel I gemäß den Ansprüchen 1 bis 5 als Monomer in der Polymerisation.
